# EUROPEAN PATENT SPECIFICATION

(11) **EP 1 501 432 B1**
(45) Date of publication and mention of the grant of the patent: **11.07.2012**
(21) Application number: 03733986.8
(22) Date of filing: 09.05.2003
(51) Int. Cl.: A61B 17/34

(54) **ADJUSTABLE BALLOON ANCHORING TROCAR**
VERSTELLBARER VERANKERUNGSBALLON FÜR TROKAR
TROCART REGLABLE POUR L'ANCRAGE D'UN BALLONNET

(30) Priority: 09.05.2002 US 379324 P
(43) Date of publication of application: 02.02.2005
(73) Proprietor: Tyco Healthcare Group LP, Mansfield, MA 02048 (US)
(72) Inventor: HEINRICH, Russell, Madison, CT 06443 (US)
(74) Representative: Soames, Candida Jane
(86) International application number: PCT/US2003/014698
(87) International publication number: WO 2003/094994

(56) References cited:
- US-A- 5 697 946
- US-A- 5 946 781

## Description

### BACKGROUND

### 1. Technical Field

The present disclosure relates to an improved balloon trocar anchoring system and, more particularly to an adjustable balloon trocar anchoring system.

### 2. Background of Related Art

Laparoscopic surgery has developed into an increasingly important and widespread surgical technique. In the past, when performing an open surgical procedure in the abdominal cavity, a large incision through the abdominal wall was required to permit entry of surgical instrumentation and viewing of the operative site. The development of the laparoscope, a small telescope utilizing fiber optic technology, now permits the surgeon to view the operative site, within the abdominal cavity, through a small incision which is only large enough for the insertion of the laparoscope. Laparoscopic surgery advantageously reduces the risk of infection to the patient and the extent of trauma to the body during surgery.

Generally, during a laparoscopic procedure, the abdominal cavity is insufflated to displace the abdominal wall from the underlying internal organs thereby permitting unrestricted access for performing the desired surgical procedure. A trocar, including a cannula sleeve and an obturator, is then used to penetrate the abdominal wall. The obturator is removed leaving the cannula sleeve in place in the abdominal wall. Instruments required to perform the surgery, such as, for example, laparoscopes, endoscopes, clip appliers, cautery devices and the like, may be inserted through the cannula sleeve. Typically, multiple trocars are utilized during a surgical procedure to provide varying access positions strategically located about the abdominal wall.

During the surgical procedure, it is desirable to secure or anchor the cannula sleeve position in the incision to prevent movement of the cannula sleeve relative to the abdominal wall, and to prevent the cannula sleeve from slipping out of the incision, causing loss of insufflation pressure from the abdominal cavity.

Prior anchors have typically employed threaded sleeves adapted to engage the abdominal wall tissue to secure the cannula sleeve in place.

Moreover, once the cannula sleeve is anchored into position, the prior art anchoring systems do not permit adjustment of the depth of the cannula sleeve. Accordingly, in circumstances where the surgeon needs to reach tissue which is remote from the puncture site for the cannula sleeve, such as in pelvic, lower colon or esophageal work, the extra length of the cannula sleeve extending outside of the patient may prevent the surgeon from reaching the desired tissue effectively shortening the instrument. You may also want to limit length of the trocar within the abdomen to give more space or to avoid organs or other instruments internally. Thus, the need exists for an adjustable anchoring cannula sleeve which will allow the anchoring device to slide along the length of the trocar, thus allowing the surgeon to set the length of the trocar inside and outside of the patient as required by the particular surgical procedure.

### SUMMARY

According to the present invention as defined by claim 1, an anchoring apparatus for use with an access sleeve, the access sleeve adapted for passage through tissue and having a lumen permitting introduction of instruments through the sleeve is provided. The anchoring apparatus includes a collar for positioning about the access sleeve, the collar defining a longitudinal axis and being adapted for movement relative to the access sleeve, and an inflatable membrane secured to the collar, the inflatable membrane adapted to be expanded to securely engage tissue and to substantially anchor the collar relative to the tissue while permitting axial or coaxial movement of the access sleeve relative to the collar. The anchoring apparatus includes a locking device for securing the position of the collar with respect to the sleeve. Preferred embodiments of the invention are defined in the dependent claims.

In one embodiment, it is envisioned that the anchoring apparatus includes a ring element coaxially mounted about an intermediate portion of the inflatable membrane and arranged to expose a portion of the inflatable membrane along at least one end of the collar. It is further envisioned that the ring element is substantially equidistant from a proximal and a distal end of the collar, and wherein the inflatable membrane is exposed along the proximal and the distal ends of the elongate collar.

According to another aspect of the present disclosure, an adjustable balloon anchoring instrument adapted for passage through tissue is provided. The anchoring instrument includes a cannula, a balloon anchoring device positionable about the cannula, the anchoring device being slidable with respect to the cannula. The instrument includes a locking device for securing the position of the balloon anchoring device with respect to the cannula. An engagement member is desirably disposed between the cannula and the anchoring device for slidably engaging the balloon anchoring device and cannula while permitting movement of the cannula and balloon anchoring device with respect to one another. It is contemplated that the engagement member is arranged to inhibit passage of fluid from between the cannula and the balloon anchoring device.

It is envisioned that the balloon anchoring device includes an elongate collar configured and adapted to slidably receive the cannula, a sleeve configured and adapted to overlie the elongate collar, wherein a first end of the sleeve is secured to a distal end of the collar and a second end of the sleeve is secured to a proximal end of the elongate collar. An elongate ring may be coaxially mounted around the sleeve, and an inflation tube in fluid communication with a space defined between the elongate collar and the sleeve may also be provided. It is further envisioned that the elongate collar has a length and wherein the elongate ring has a length which is shorter than the length of the elongate collar, wherein a portion of the sleeve is exposed at least along one end of the elongate collar.

In one embodiment, it is envisioned that the balloon anchoring device includes an elongate collar configured and adapted to slidably receive the cannula and a distal balloon secured to a distal end of the elongate collar, a proximal balloon secured to a proximal end of the aid elongate collar. An inflation tube in fluid communication with the proximal balloon and/or the distal balloon is desirable. An inflation lumen may be formed through the elongate collar in communication with the inflation tube. Preferably, the proximal and distal toroidal balloons are spaced from one another. It is envisioned that the distal balloon and the proximal balloon are spaced from one another so as to engage tissue therebetween. The space between the proximal and the distal balloons may be less than a thickness of the tissue. In a preferred embodiment, the proximal and distal balloons are movable with respect to one another on the cannula.

In another embodiment, it is envisioned that the balloon anchoring device includes an elongate collar configured and adapted to slidably receive the cannula, a balloon secured to a distal end of the elongate collar, an inflation tube in fluid communication with the balloon via an inflation lumen formed through the elongate collar. A retention collar having an aperture therethrough for positioning about the elongate collar may also be provided.

According to yet another embodiment of the present disclosure, an anchoring device for use with a surgical instrument adapted for percutaneous access through tissue is disclosed. The anchoring device includes a collar having a distal end portion, an intermediate portion and a proximal end portion, the collar defining a lumen for passage of the surgical instrument therethrough. A sleeve secured to an outer surface of the collar, the sleeve being adapted to expand in a radial direction to securely engage the tissue and substantially anchor the collar relative to the tissue while permitting movement of the surgical instrument relative to the collar. A locking device is included for securing the position of the collar with respect to the surgical instrument.

It is further envisioned that the anchoring device includes a ring element coaxially mounted about the intermediate portion of the collar and arranged to expose a portion of the sleeve near both the distal and proximal end portions of the collar. It is envisioned that the ring may be radially expandable.

According to yet another aspect of the present disclosure, an anchoring cannula is provided including a cannula, a collar having a distal end and a proximal end and a lumen for slidably receiving the cannula, a radially expandable member attached to the distal end of the collar, a retention collar attached to the proximal end of the collar, and an engagement member disposed between the cannula and the collar for permitting movement of the cannula relative to the collar. The engagement member may be arranged to inhibit the passage of fluid from between the cannula and the collar.

Various additional features of novelty which characterize the disclosure, are pointed out with particularity in the claims annexed hereto and forming a part of this disclosure.

### BRIEF DESCRIPTION OF THE DRAWINGS

The accompanying drawings, which are incorporated in and constitute a part of this specification, illustrate embodiments of the invention and, together with a general description of the invention given above, and the detailed description of the embodiments given below, serve to explain the principles of the invention.
FIG. 1 is an elevational view of a prior art balloon anchor in place on a trocar sleeve, with the balloon of the anchor in a deflated condition and a trocar extended through the trocar sleeve in the process of forming a puncture opening in a tissue layer;
FIG. 2 is an elevational view similar to that of FIG. 1, showing the trocar sleeve fully extended through the puncture opening, with the balloon inflated and the trocar removed from the trocar sleeve;
FIG. 3 is a cross-sectional view taken through lines 3 -- 3 of FIG. 1;
FIG. 4 is a cross-sectional view taken through lines 4 -- 4 of FIG. 2;
FIG. 5 is a partial perspective view of an adjustable balloon anchoring apparatus in accordance with an embodiment of the present invention;
FIG. 6 is an elevational view of an adjustable balloon anchoring apparatus, in accordance with the embodiment of FIG. 5 of the present invention;
FIG. 7 is a cross-sectional elevational view taken along lines 7--7 of FIG. 6;
FIG. 8 is a cross-sectional elevational view of an adjustable balloon anchoring apparatus in accordance with another embodiment of the present invention;
FIG. 9 is a cross-sectional elevational view of an adjustable balloon anchoring apparatus in accordance with still another embodiment of the present invention;
FIG. 10 is a cross-sectional view of a balloon anchoring apparatus in accordance with a further embodiment of the invention;
FIG. 11 is a cross-sectional view of a balloon anchoring apparatus in accordance with another embodiment of the invention; and
FIG. 12 is a cross-sectional view taken along line 12-12 in FIG. 11.

### BRIEF DESCRIPTION OF PREFERRED EMBODIMENTS

Preferred embodiments of the presently disclosed balloon anchoring trocar will now be described in detail with reference to the drawing figures wherein like reference numerals identify similar or identical elements. In the drawings and in the description which follows, the term "proximal", as is traditional will refer to the end of the surgical device or instrument of the present disclosure which is closest to the operator, while the term "distal" will refer to the end of the device or instrument which is furthest from the operator.

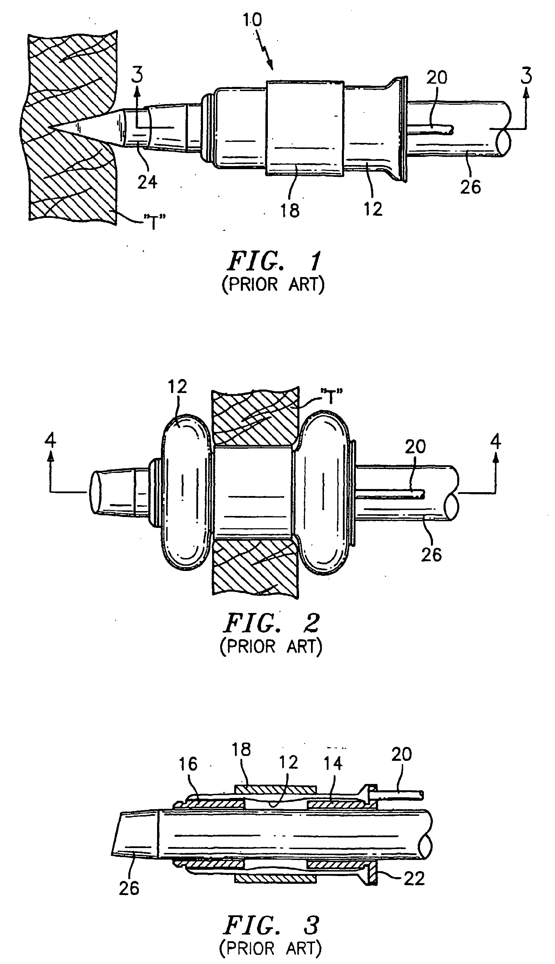

FIGS. 1-4 illustrate a prior art balloon anchoring trocar, as disclosed in commonly assigned U.S. Pat. No. 5,697,946 to Hopper et al. The preamble of claim 1 is based as this disclosure. As seen in FIGS. 1-4, the balloon anchoring trocar is generally designated as 10. Balloon anchoring trocar 10 includes a balloon 12, spaced apart proximal and distal rings 14 and 16, respectively, an intermediate ring 18 disposed around balloon 12 and between proximal and distal rings 14 and 16, and an inflation tube 20 in fluid communication with the interior of balloon 12 through a flange 22 on proximal ring 14.

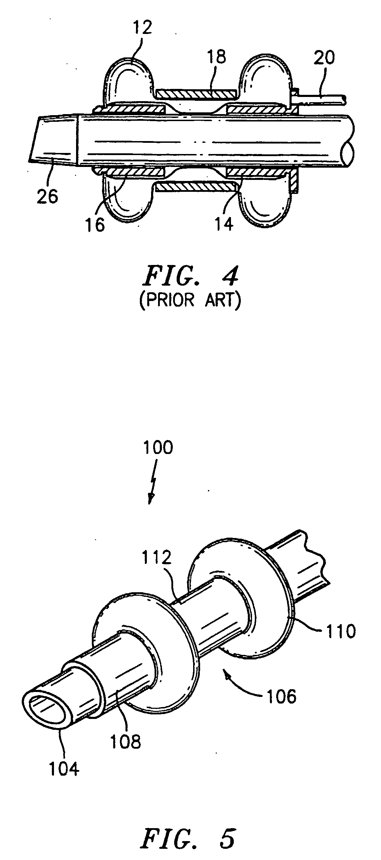

In certain embodiments disclosed in the '946 Patent, balloon 12 is deflated and a sharp tipped trocar 24 extends through a cannula sleeve 26 into piercing engagement with a layer of living tissue "T". Once fully extended through tissue "T", balloon 12 is inflated as shown in FIG. 2 to expand to either side of tissue "T". As so expanded, balloon 12 forms barriers on either side of tissue "T". Expansion of balloon 12 within the thickness of tissue "T" is prevented by intermediate ring 18, while the space between distal and proximal rings 14 and 16 permits the interior wall of balloon 12 to expand into gripping engagement with the outer surface of sleeve 26, as shown in FIG. 4. Thus, sleeve 26 is anchored against movement into or out of pierced tissue "T".

An embodiment of an adjustable apparatus is shown in FIGS. 5-7, wherein like reference numerals identify similar or identical elements throughout the several views. The adjustable anchoring apparatus 100 has an independent sliding apparatus 106 that receives a cannula sleeve 104. In certain embodiments, cannula sleeve 104 receives a sharp tipped trocar 102. In other embodiments, cannula sleeve 104 receives a blunt or relatively blunt obturator (not shown).

Sliding apparatus 106 includes an annular elongate collar 108 configured and adapted to surround cannula sleeve 104 and an expandable sleeve 110 surrounding collar 108. Expandable sleeve 110 is desirably a membrane. One end of sleeve 110 is secured to a distal end of collar 108 and a second end of sleeve 110 is secured to a proximal end of collar 108. Preferably sleeve 110 is secured to collar 108 with an adhesive, however, it is envisioned that sleeve 110 can be secured to collar 108 using any other known method, such as, for example, the use of a cord to tie the ends of sleeve 110 down to collar 108.

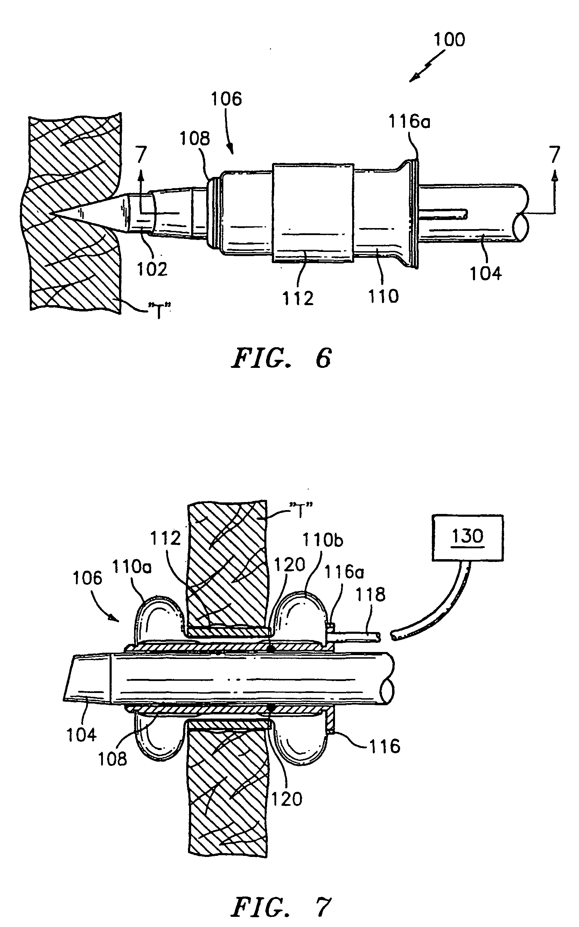

Sliding apparatus 106 preferably further includes an elongate ring 112 disposed around sleeve 110. Preferably, elongate ring 112 has a length which is shorter than the length of collar 108. In this manner, as seen in FIG. 7, when a fluid (e.g., air, oxygen, CO₂, saline, water, etc.) is injected into the space between sleeve 110 and collar 108, the portions of sleeve 110 not covered by ring 112 will expand radially outward. Preferably, ring 112 is located between the distal and proximal ends of collar 108 so that a distal and proximal end of sleeve 110 is left uncovered by ring 112. Accordingly, when the space between elastic sleeve 110 and collar 108 is inflated, a pair of doughnut shaped barriers 110a, 110b will form at either end of ring 112.

Preferably, the length of ring 112 is selected to be smaller than the thickness of the body tissue through which the apparatus is to pass. In this manner, when the space between sleeve 110 and collar 108 is inflated, the body tissue will be squeezed between barriers 110a and 110b of radially expanding sleeve 110 thereby securing and/or anchoring sliding apparatus 106 to the body tissue.

Preferably ring 112 is a solid ring made from a surgical grade metal or polymer. In further embodiments, ring 112 may include an expandable structure. It is envisioned that ring 112 can be an elongate elastomeric split ring having a pair of overlapping ends (not shown). In this manner, as the space between sleeve 110 and collar 108 is inflated, the ends of split ring will slide over one another thereby taking up and filling the space of the opening in the body. As such, the escape of insufflation gas through the space between sliding apparatus 106 and body tissue "T" is reduced.

Preferably, an inflation tube 118 is connected to the space between sleeve 110 and collar 108. In the embodiment shown, a flange 116 is provided at the proximal end of collar 108 to which an inflation tube 118 is coupled. Inflation tube 118 inter-connects the space between sleeve 110 and collar 108 with a source of inflation fluid 130. The proximal end of sleeve 110 is desirably secured to a rim 116a of flange 116 so that tube 118 communicates with the space to be inflated.

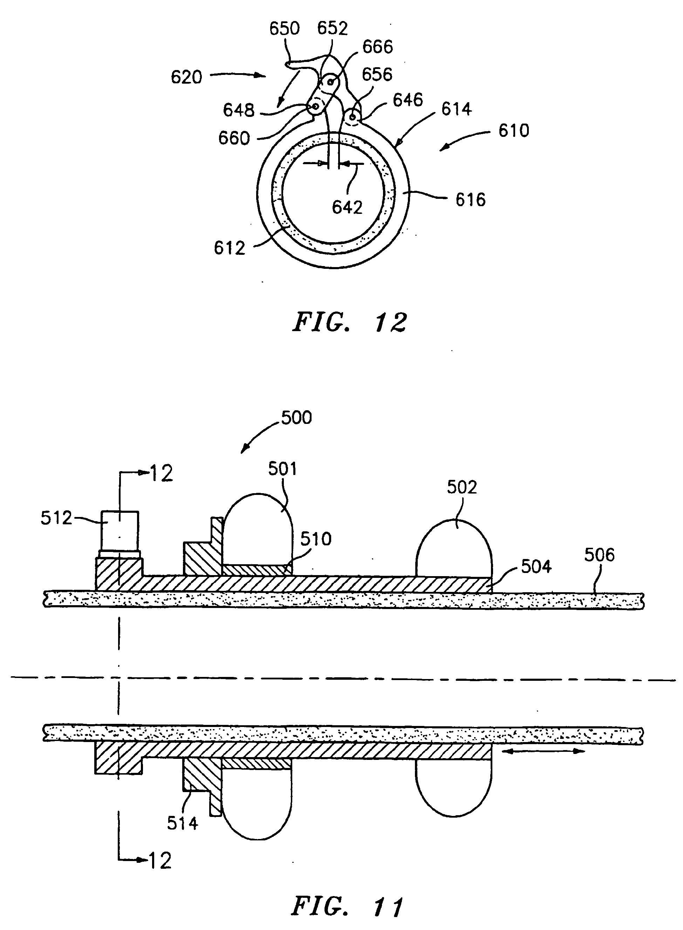

Sliding apparatus 106 further includes a locking device for securing the position of collar 108 on cannula sleeve 104. Any locking device known in the art may be used. For example, locking devices disclosed in certain embodiments of WO 02/096307 , may be used. The locking devices discussed below in connection with FIG. 12 may be used.

An elastomeric O-ring 120 is also desirably disposed between collar 108 and cannula sleeve 104. Desirably, O-ring 120 provides a seal between collar 108 and cannula sleeve 104, which seal prevents the escape of insufflation gas through the space between collar 108 and cannula sleeve 104. O-ring 120 is also preferably arranged to slidably engage collar 108 and cannula sleeve 104.

In use, sliding apparatus 106 is simply slipped over the desired selected surgical instrument such as, for example, cannula sleeve 104. In a preferred embodiment, cannula sleeve 104 is part of an apparatus that includes a trocar or obturator received by the cannula sleeve. As seen in FIG. 7, once trocar 102 is fully extended through body tissue "T" and sliding anchor 106 positioned such that collar 108 extends across the width of body tissue "T" and ring 112 is positioned substantially in line with body tissue "T", the space between sleeve 110 and collar 108 is inflated thereby forming barriers on either side of body tissue "T". Radial expansion of the portion of sleeve 110 in line with body tissue "T" is prevented by ring 112, however, sleeve 110 is permitted to radially expand into gripping engagement with the outer and inner surface of body tissue "T" at locations proximal and distal of ring 112. Thus, sliding apparatus 106 is secured against movement into and/or out of pierced body tissue "T". In addition, cannula sleeve 104 is advantageously free to axially move, slide and/or telescope through sliding apparatus 106 thereby allowing the surgeon to set the length of cannula sleeve 104 inside and outside of the patient as may be required. The locking device is activated to engage cannula sleeve 104 for locking the position of the anchoring apparatus with respect to cannula sleeve 104 after the surgeon has set the position of the anchoring apparatus with respect to cannula sleeve 104. Upon completion of the surgical procedure, the inflating medium (e.g., air) is released from sliding apparatus 106, thus deflating sleeve 110 and permitting removal of sliding apparatus 106 and trocar 102 from body tissue "T".

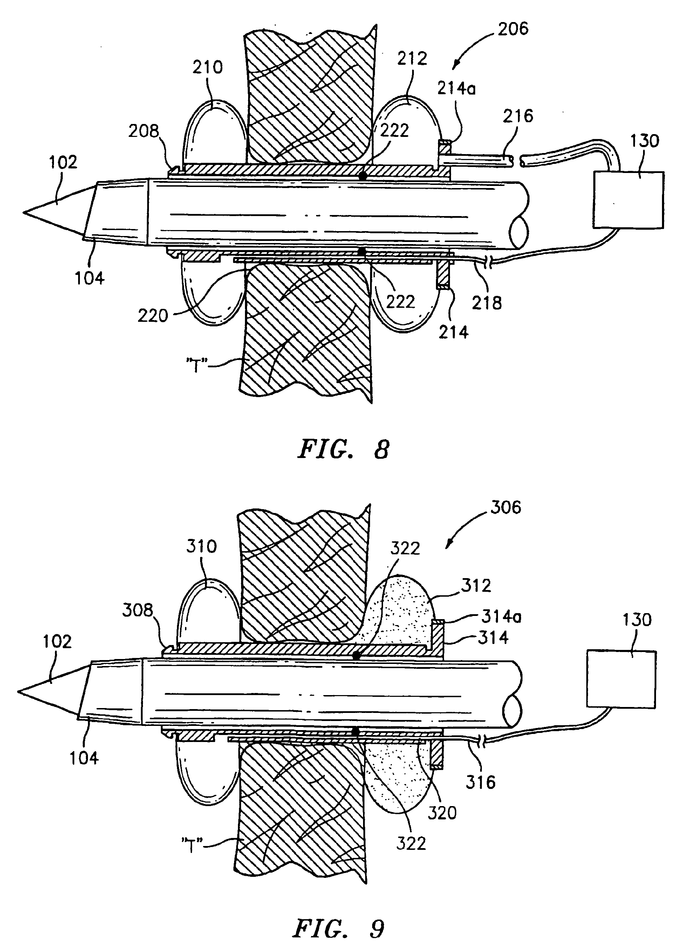

An alternative embodiment of an anchoring apparatus in accordance with the present disclosure is shown in FIG. 8. The anchoring apparatus has a sliding apparatus 206, which includes an annular elongate collar 208 configured and adapted to slidably receive cannula sleeve 104. The anchoring apparatus has a distal anchoring device with an expandable sleeve. In the embodiment shown, the distal anchoring device includes a first toroidal balloon 210 secured to the distal end of collar 208, and a second anchoring device including a second toroidal balloon 212 secured to a proximal end of collar 208. In other embodiments, second toroidal balloon 212 may include other expandable structures, or may include a foam collar disposed on elongate collar 208. Preferably, first balloon 210 and second balloon 212 are spaced from one another. It is preferred that first and second balloons 210 and 212 are adhesively secured to collar 208, however, other known methods of securing balloons 210, 212 to collar 208 are contemplated. Although the balloons shown have a toroidal shape, other shapes may be used.

Preferably, the anchoring apparatus includes at least one inflation tube. Collar 208 may include a flange 214 formed along a proximal end thereof for coupling with a source of inflation. A proximal end of second balloon 212 is preferably secured to flange 214 so that the inflation tube communicates with second balloon 212. In the embodiment shown, a first inflation tube 216 is operatively coupled to flange 214 and is in fluid communication with second balloon 212. A second inflation tube 218 may be operatively coupled to flange 214 and is in fluid communication with an inflation lumen 220 extending from flange 214 to first balloon 210. First and second inflation tubes 216 and 218 interconnect second and first balloons 212 and 210, respectively, with a source of inflation fluid 130. While first and second inflation tubes have been disclosed, it is envisioned that a single inflation tube can be operatively and fluidly coupled to a single lumen provided in sliding apparatus 206, which single lumen extends between both the first and the second balloons and thus permits the first and the second balloons to be inflated simultaneously via the single inflation tube. The inflation lumens disclosed herein may also include a lumen defined in the wall of collar 208.

Sliding apparatus 206 further includes a locking device for securing the position of collar 108 on cannula sleeve 104. Any locking device known in the art and/or disclosed herein may be used.

Sliding apparatus 206 further desirably includes an elastomeric O-ring 222 disposed between collar 208 and cannula sleeve 104. O-ring 222 provides a seal between collar 208 and cannula sleeve 104, which seal prevents the escape of insufflation gas through the space between collar 208 and cannula sleeve 104.

In use, anchoring apparatus 206 is simply slipped over the desired selected surgical instrument, e.g., cannula sleeve 104. In certain preferred embodiments, cannula sleeve 104 receives a trocar 102 or an obturator. As shown in FIG. 8, once trocar 102 is fully extended through body tissue "T" and sliding apparatus 206 positioned such that first and second balloons 210, 212 are disposed on either side of body tissue "T", first and second balloons 210, 212 are inflated thereby forming barriers on either side of body tissue "T". First and second balloons 210, 212 are inflated until balloons 210, 212 are in gripping engagement with the outer and inner surface of body tissue "T". Thus, sliding apparatus 206 is secured against movement into and/or out of pierced body tissue "T". Cannula sleeve 104 is free to axially move, slide or telescope through sliding apparatus 206 as discussed herein above. The surgeon secures the position of the anchoring device with respect to the cannula sleeve using the locking device. Upon completion of the surgical procedure, at least first balloon 210 is deflated so that sliding apparatus 206 and trocar 102 can be withdrawn from body tissue "T".

An alternative embodiment of an anchoring device in accordance with the present disclosure is shown in FIG. 9. The anchoring device has a sliding apparatus 306, which includes an annular elongate collar 308, and a balloon 310 secured to the distal end of collar 308, substantially as discussed above. The proximal anchoring device includes a retention collar 312 (desirably in the form of a toroid) having an aperture therethrough for positioning about a proximal end of collar 308. Retention collar 312 is desirably slidably moveable along elongate collar 308 to prevent inadvertent movement of sliding apparatus 306 into or out of the abdominal cavity. In other embodiments, retention collar 312 may be attached to collar 308. Retention collar 312 can be made from silicone, foam, or any other resilient material. If slidable on collar 308, the anchoring device desirably includes a locking device for securing the position of retention collar 312 with respect to collar 308.

In the present embodiment, collar 308 includes a flange 314 formed along a proximal end thereof. Flange 314 is configured and dimensioned to prevent retention collar 312 from sliding off of the proximal end of elongate collar 308. An inflation tube 316 is operatively coupled to flange 314 and is in fluid communication with an inflation lumen 320 extending from flange 314 to balloon 310. Inflation tube 316 interconnects balloon 310 with a source of inflation fluid 130. Other means of inflating the distal anchoring balloon may also be used.

The anchoring device includes a locking device disposed between collar 308 and cannula sleeve 104. Any locking device known in the art and/or disclosed herein may be used. Sliding apparatus 306 further desirably includes an elastomeric O-ring 322 disposed between collar 308 and cannula sleeve 104 to prevent the escape of insufflation gas through these components.

In use, sliding apparatus 306 is simply slipped over the desired selected surgical instrument, e.g., cannula sleeve 104. In a preferred embodiment, cannula sleeve 104 receives a trocar or obturator. As seen in FIG. 9, once trocar 102 is fully extended through body tissue "T" and sliding apparatus 306 positioned such that retention collar 312 is in contact with the outer surface of body tissue "T", balloon 310 is inflated against the inner surface of body tissue "T", thereby preventing sliding apparatus 306 from being withdrawn through body tissue "T". Balloon 310 and retention collar 312 cooperate together to fixedly position sliding apparatus 306 with respect to body tissue "T" while permitting cannula sleeve 104 to freely move axially move or telescope within sliding apparatus 306 thereby allowing the surgeon to set the length of cannula sleeve 104 inside and outside of the patient as required by the particular surgical procedure. The locking device is utilized to secure the position of collar 308 with respect to cannula sleeve 104. Upon completion of the surgical procedure, balloon 310 is deflated for the removal of sliding apparatus 306 and trocar 102 from body tissue "T".

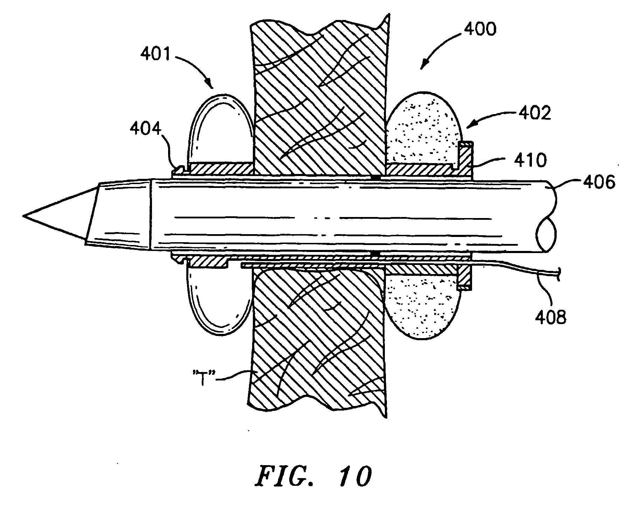

In a further embodiment shown in FIG. 10, anchoring apparatus 400 includes a distal anchoring device 401 that is separately movable from proximal anchoring device 402. In the embodiment shown, distal anchoring device 401 includes a balloon mounted on a first collar 404 that slidably receives a cannula sleeve 406 or other instrument. First collar 404 desirably includes a locking device for securing the position of first collar 404 with respect to cannula sleeve 406, or other instruments. A proximal anchoring device 402 includes an expandable anchoring device, or retention collar, or skin seal. In a preferred embodiment, proximal anchoring device 402 includes a retention collar having a locking device for securing the position of the retention collar with respect to the cannula sleeve. The retention collar may include a second collar 410 that slidably receives cannula sleeve 406. Anchoring apparatus 400 includes an inflation tube 408 or other inflation devices for delivering an inflation fluid to distal anchoring device 401. Second collar 410 may define a passage in which inflation tube 408 extends.

In use, the anchoring device is inserted into the incision with or before the instrument is inserted into the cannula sleeve. The anchoring device is deployed desirably after adjusting the relative positions of the anchoring device and cannula sleeve. The proximal anchoring device is then advanced to engage the abdominal wall.

The independently movable distal anchoring device 401 and proximal anchoring device 402 are adjustable for engaging abdominal walls of varying thickness and/or for adjusting the degree to which the anchoring devices squeeze the abdominal wall.

In a further embodiment shown in FIG. 11, anchoring apparatus 500 includes a distal anchoring device 501 that is separately moveable from a proximal anchoring device 502. In the embodiment shown, proximal anchoring device 502 includes a balloon mounted on a first collar 504 that slidably receives a cannula sleeve 506 or other instrument. First collar 504 desirably includes a locking device 512 for securing the position of first collar 504 with respect to cannula sleeve 506, or other instrument. Locking device 512 may include any locking device known in the art and/or disclosed herein. In alternative embodiments, proximal anchoring device 502 may include a retention collar of silicone, foam or any other resilient material.

Distal anchoring device 501 desirably includes an expandable anchoring device. Distal anchoring device 501 may include a second collar 510 that slidably receives first collar 504. In a preferred embodiment, distal anchoring device 501 includes a balloon mounted on second collar 510 and has a locking device 514 for securing the position of distal anchoring device 501 with respect to proximal anchoring device 502. The locking device is desirably formed on second collar 510 and may include any locking device known in the art and/or disclosed herein. Anchoring apparatus 500 includes one or more inflation tubes or other inflation device, as discussed above, for delivering an inflation fluid to distal anchoring device 501 and/or proximal anchoring device 502. For example, first collar 504 may define a passage in which the inflation tube extends.

The anchoring devices, retention collars, and/or balloons desirably include a locking device for securing the position of the anchoring apparatus with respect to the cannula sleeve and for facilitating adjustment of the anchoring apparatus on the cannula sleeve. Referring to FIG. 12, an anchoring device 610 generally includes a frame 614 including a locking collar 616. A latch assembly 620 is provided on locking collar 616 to secure anchoring device 610 at a location along a cannula sleeve 612 as described herein. A foam pad or balloon or other anchoring member is affixed to locking collar 610 and is compressible against the abdominal wall to provide a secure seal.

As shown locking collar 616 is not completely circumferential but defines a split 642 which allows locking collar 616 to be slightly flexible and compressible against cannula sleeve 612. Mounting projections 646 and 648 are formed on either side of split 642. Latch assembly 620 is of the "over center clamp" design and generally includes a lever 650 and a cam bar 652. Lever 650 is pivotally connected at a first end to mounting projection 646 by a pin 656 and cam bar 652 is pivotally connected at a first end 658 to mounting projection 648 by a pin 660. A second end of cam bar 652 is pivotally connected to a central portion of lever 650 by a pin 666.

The clamping action of latch assembly 620 will now be described. When lever 650 is in an open position, the distance between mounting projections 646 and 648 are at maximum and locking collar 616 is free to slide along cannula sleeve 612. As lever 650 is rotated, cam bar 652 moves through an arc and drives mounting projection 648 towards mounting projection 646 to compress against cannula sleeve 612.

In another alternative locking device, a clamping band, similar to the locking collar 616 is split and includes mounting projections at one end of the clamping band and an extension extending from an opposite end of clamping band. The extension terminates in a cross-wise pin that engages recesses formed in a latch body. The latch body is pivotable to draw the extension closed towards the opposed end of band thereby ensuring a secure seal about an associated cannula sleeve. The anchoring devices, retention collars and/or balloons may include the locking devices discussed above, or the locking devices may be separately provided on the anchoring apparatus. Furthermore, in any of the embodiments discussed above, the locking device may include an O-ring for securing the position of the anchoring apparatus with respect to the cannula sleeve using the friction between the O-ring, anchoring apparatus and cannula sleeve.

The locking devices discussed above may be as described in certain embodiments of WO 02/096307 .

In further embodiments, the expandable sleeve may include a balloon, sponge, or malecot structure or onion, resilient member or bellows. Both the distal and proximal anchoring devices, as well as the retention collar may include a balloon, sponge, malecot structure, onion (which may have resilient arms with living hinges), resilient members, or bellows, or any combination of the foregoing. The balloon may be formed by a membrane enclosing an inflatable interior, or by a membrane forming an inflatable collar or other surface. Furthermore, the position of the anchoring apparatus may be adjusted and locked into position with respect to a cannula sleeve or other instrument prior to or after insertion of the apparatus into the body.

It will be understood that various modifications may be made to the embodiments disclosed herein. Therefore, the above description should not be construed as limiting, but merely as exemplifications of preferred embodiments. Those skilled in the art will envision other modifications within the scope of the claims appended hereto.

## Claims

1. An anchoring apparatus (100, 206, 306, 400, 500) for use with an access sleeve (104, 506), the access sleeve adapted for passage through tissue and having a lumen permitting introduction of instruments through the sleeve, the anchoring apparatus comprising:
a collar (108, 208, 308, 404, 504) for positioning about the access sleeve, the collar defining a longitudinal axis; an inflatable membrane (110, 210, 212, 310, 401, 402, 501, 502) secured to the collar, **characterised in that**:
the collar is a contiguous annular elongate collar to said access sleeve adapted for slidable movement relative to the access sleeve;
the inflatable membrane surrounds and is secured to the collar and is expandable to securely engage tissue and to substantially anchor the collar relative to the tissue while permitting axial or coaxial movement of the access sleeve relative to the collar; and
a locking device (512) secures the position of the collar with respect to the sleeve.

2. The anchoring apparatus according to claim 1, further including a ring element (112) coaxially mounted about an intermediate portion of the inflatable membrane and arranged to expose a portion of the inflatable membrane along one end of the collar.

3. The anchoring apparatus according to claim 2, wherein the ring element is substantially equidistant from a proximal and a distal end of the collar, and wherein the inflatable membrane is exposed along the proximal and the distal ends of the elongate collar.

4. The anchoring apparatus according to claim 1, wherein the access sleeve is a cannula (104) and the inflatable membrane is a balloon anchoring device positionable about the cannula, the anchoring apparatus being slidable with respect to the cannula; and the locking device secures the position of the balloon anchoring device with respect to the cannula.

5. The anchoring apparatus according to claim 4, further comprising an engagemement member (120, 222, 322) disposed between the cannula and the anchoring device for slidably engaging the balloon anchoring device and cannula while permitting movement of the cannula and balloon anchoring device with respect to one anchor.

6. The anchoring apparatus according to claim 5, wherein the engagement member is arranged to inhibit the passage of fluid between the cannula and the balloon anchoring device.

7. The anchoring apparatus according to claim 4, wherein:
the collar is configured and adapted to slidably receive the cannula;
the inflatable membrane is a sleeve configured and adapted to overlie the elongate collar, wherein a first end of the sleeve is secured to a distal end of the elongate collar and a second end of the sleeve is secured to a proximal end of the elongate collar.

8. The anchoring apparatus according to claim 7, further comprising:
an elongate ring (112) coaxially mounted around the elastic sleeve; and
an inflation tube (118, 218, 316, 408) in fluid communication with a space defined between the elongate collar and the sleeve.

9. The anchoring apparatus according to claim 8, wherein the elongate collar has a length and wherein the elongate ring has a length which is shorter than the length of the elongate collar, wherein a portion of the sleeve is exposed at least along one end of the elongate collar.

10. The anchoring apparatus according to claim 4, wherein:
the collar is configured and adapted to slidably receive the cannula;
the inflatable membrane is a distal balloon (210, 401, 501) secured to a distal end of the elongate collar; and the balloon anchoring device comprises a proximal balloon (212, 502) secured to a proximal end of the elongate collar.

11. The anchoring apparatus according to claim 10, further comprising a first inflation tube (216) in fluid communication with the proximal balloon; and
a second inflation tube (218) in fluid communication with the distal balloon via an inflation lumen formed through the elongate collar.

12. The anchoring apparatus according to claim 11, wherein the elongate collar defines an inflation lumen (220, 320) in communication with the inflation tube.

13. The adjustable balloon anchoring instrument according to claim 10, wherein the distal balloon and the proximal balloon are spaced from one another so as to engage tissue therebetween.

14. The anchoring apparatus according to claim 4, wherein:
the collar is an elongate collar configured and adapted to surround the cannula;
the inflatable membrane is a balloon secured to a distal end of the elongate collar; and
the anchoring device comprises an inflation tube in fluid communication with the balloon via an inflation lumen formed through the elongate collar.

15. The anchoring apparatus according to claim 14, further comprising a retention collar (312, 402) having an aperture therethrough for positioning about the elongate collar.

16. The anchoring apparatus according to claim 1, which is an anchoring device for use with a surgical instrument providing the access sleeve, the instrument adapted for percutaneous access through tissue, wherein:
the collar has a distal end portion, an intermediate portion and a proximal end portion, the collar defining a lumen for passage of the surgical instrument therethrough;
the inflatable membrane is a sleeve secured to an outer surface of the collar, the sleeve being adapted to expand in a radial direction to securely engage the tissue and substantially anchor the collar relative to the tissue while permitting movement of the surgical instrument relative to the collar; and
the locking device is for securing the position of the collar with respect to the surgical instrument.

17. The anchoring apparatus of claim 16, further including a ring element (112) coaxially mounted about the intermediate portion of the collar and arranged to expose a portion of the sleeve near both the distal and proximal end portions of the collar.

18. An anchoring apparatus according to claim 1, wherein the access sleeve is a cannula, wherein:
the collar has a distal end and a proximal end and a lumen for slidably receiving the cannula;
the inflatable membrane is a radially expandable member attached to the distal end of the collar; the anchoring cannula comprises:
a retention collar (12, 410) attached to the proximal end of the collar; and
an engagement member (120, 222, 322) disposed between the cannula and the collar for permitting movement of the cannula relative to the collar.

19. The anchoring apparatus of claim 18, wherein the engagement member is arranged to inhibit the passage of fluid from between the cannula and the collar.

20. An anchoring apparatus according to claim 1, wherein the access sleeve is a cannula, wherein:
the collar is a distal anchoring device (401) slidably receiving the cannula, the distal anchoring device comprising an expandable sleeve which provides the inflatable membrane; the locking device is for securing the position of the distal anchoring device with respect to the cannula; the
anchoring instrument comprising:
a proximal anchoring device (402) disposed on the cannula at a position proximal of the distal anchoring device.

21. The anchoring apparatus of claim 20, wherein the proximal anchoring device comprises a foam collar (402).

22. The anchoring apparatus according to claim 20, wherein the proximal anchoring device slidably receives the cannula and further comprises a locking device for securing the position of the proximal anchoring device with respect to the cannula.

23. The anchoring apparatus of claim 20, wherein the cannula defines a lumen and further suited for an instrument received in the lumen.

24. The anchoring apparatus of claim 20, wherein the instrument comprises a trocar (102).

## Patentansprüche

1. Verankerungsgerät (100, 206, 306, 400, 500) zur Verwendung mit einer Zugangsmuffe (104, 506), wobei die Zugangsmuffe ausgelegt ist zum Durchgang durch Gewebe und ein Lumen aufweist, das die Einführung von Instrumenten durch die Muffe ermöglicht, wobei das Verankerungsgerät Folgendes umfasst:
eine Manschette (108, 208, 308, 404, 504) zur Positionierung um die Zugangsmuffe, wobei die Manschette eine Längsachse definiert; wobei eine aufblasbare Membran (110, 210, 212, 310, 401, 402, 501, 502) an die Manschette befestigt ist, **dadurch gekennzeichnet, dass**:
die Manschette eine ringförmige verlängerte Manschette ist, die an der Zugangsmuffe anliegt, ausgelegt zur gleitenden Bewegung mit Bezug auf die Zugangsmuffe;
die aufblasbare Membran die Manschette umgibt und daran befestigt ist und expandiert werden kann, um sicher in das Gewebe einzugreifen und im Wesentlichen die Manschette mit Bezug auf das Gewebe zu verankern, während sie eine axiale oder koaxiale Bewegung der Zugangsmuffe mit Bezug auf die Manschette ermöglicht; und
eine Verschlussvorrichtung (512) die Position der Manschette mit Bezug auf die Muffe befestigt.

2. Verankerungsgerät nach Anspruch 1, weiter umfassend ein Ringelement (112), das koaxial um einen Zwischenabschnitt der aufblasbaren Membran montiert ist und angeordnet ist, um einen Abschnitt der aufblasbaren Membran entlang eines Endes der Manschette freizulegen.

3. Verankerungsgerät nach Anspruch 2, wobei das Ringelement im Wesentlichen äquidistant von einem proximalen und einem distalen Ende der Manschette ist, und wobei die aufblasbare Membran entlang dem proximalen und dem distalen Ende der verlängerten Manschette freigelegt ist.

4. Verankerungsgerät nach Anspruch 1, wobei die Zugangsmuffe eine Kanüle (104) ist und die aufblasbare Membran eine Ballonverankerungsvorrichtung ist, die um die Kanüle positioniert werden kann, wobei das Verankerungsgerät mit Bezug auf die Kanüle gleitbar ist; und die Verschlussvorrichtung die Position der Ballonverankerungsvorrichtung mit Bezug auf die Kanüle befestigt.

5. Verankerungsgerät nach Anspruch 4, weiter umfassend eine Eingriffselement (120, 222, 322), das zwischen der Kanüle und der Verankerungsvorrichtung angebracht ist, zum gleitbaren Eingriff der Ballonverankerungsvorrichtung und der Kanüle, während es die Bewegung der Kanüle und der Ballonverankerungsvorrichtung mit Bezug auf einen Anker ermöglicht.

6. Verankerungsgerät nach Anspruch 5, wobei das Eingriffsglied angeordnet ist, um den Durchgang von Fluid zwischen der Kanüle und der Ballonverankerungsvorrichtung zu verhindern.

7. Verankerungsgerät nach Anspruch 4, wobei:
die Manschette konfiguriert und ausgelegt ist, um die Kanüle gleitbar aufzunehmen;
die aufblasbare Membran eine Muffe ist, die konfiguriert und ausgelegt ist, um über der verlängerten Manschette zu liegen, wobei ein erstes Ende der Muffe an ein distales Ende der verlängerten Manschette befestigt ist und ein zweites Ende der Muffe an ein proximales Ende der verlängerten Manschette befestigt ist.

8. Verankerungsgerät nach Anspruch 7, weiter umfassend:
einen verlängerten Ring (112), der koaxial um die elastische Muffe montiert ist; und
ein Aufblasrohr (118, 218, 316, 408) in Fluidverbindung mit einem Raum, der zwischen der verlängerten Manschette und der Muffe definiert ist.

9. Verankerungsgerät nach Anspruch 8, wobei die verlängerte Manschette eine Länge aufweist, und wobei der verlängerte Ring eine Länge aufweist, die geringer als die Länge der verlängerten Manschette ist, wobei ein Abschnitt der Muffe mindestens entlang eines Endes der verlängerten Manschette freigelegt ist.

10. Verankerungsgerät nach Anspruch 4, wobei:
die Manschette konfiguriert und ausgelegt ist, um die Kanüle gleitbar aufzunehmen;
die aufblasbare Membran ein distaler Ballon (210, 401, 501) ist, der an ein distales Ende der verlängerten Manschette befestigt ist;
und die Ballonverankerungsvorrichtung einen proximalen Ballon (212, 502) umfasst, der an ein proximales Ende der verlängerten Manschette befestigt ist.

11. Verankerungsgerät nach Anspruch 10, weiter umfassend ein erstes Aufblasrohr (216) in Fluidverbindung mit dem proximalen Ballon; und
ein zweites Aufblasrohr (218) in Fluidverbindung mit dem distalen Ballon durch ein Aufblaslumen, das durch die verlängerte Manschette gebildet ist.

12. Verankerungsgerät nach Anspruch 11, wobei die verlängerte Manschette ein Aufblaslumen (220, 320) in Verbindung mit dem Aufblasrohr definiert.

13. Einstellbares Ballonverankerungsinstrument nach Anspruch 10, wobei der distale Ballon und der proximale Ballon voneinander beanstandet sind, um Gewebe dazwischen einzugreifen.

14. Verankerungsgerät nach Anspruch 4, wobei:
die Manschette eine verlängerte Manschette und ausgelegt ist, um die Kanüle zu umgeben;
die aufblasbare Membran ein Ballon ist, der an ein distales Ende der verlängerten Manschette befestigt ist; und
die Verankerungsvorrichtung ein Aufblasrohr in Fluidverbindung mit dem Ballon durch ein Aufblaslumen umfasst, das durch die verlängerte Manschette gebildet ist.

15. Verankerungsgerät nach Anspruch 14, weiter umfassend eine Rückhaltemanschette (312, 402) mit einer Öffnung dadurch zur Positionierung um die verlängerte Muffe.

16. Verankerungsgerät nach Anspruch 1, wobei es sich um eine Verankerungsvorrichtung zur Verwendung mit einem chirurgischen Instrument handelt, die die Zugangsmuffe bereitstellt, wobei das Instrument für den perkutanen Zugang durch das Gewebe ausgelegt ist, wobei:
die Manschette einen distalen Endteil aufweist, einen Zwischenteil und einen proximalen Endteil, wobei die Manschette ein Lumen für den Durchgang des chirurgischen Instruments dadurch definiert;
die aufblasbare Membran eine Muffe ist, die an eine äußere Fläche der Manschette befestigt ist, die Muffe ausgelegt ist, um in einer radialen Richtung zu expandieren, um das Gewebe sicher einzugreifen und im Wesentlichen die Manschette mit Bezug auf das Gewebe zu verankern, während sie die Bewegung des chirurgischen Instruments mit Bezug auf die Manschette ermöglicht; und
die Verschlussvorrichtung zur Befestigung der Position der Manschette mit Bezug auf das chirurgische Instrument dient.

17. Verankerungsgerät nach Anspruch 16, weiter umfassend ein Ringelement (112), das koaxial um den Zwischenabschnitt der Manschette montiert ist und angeordnet ist, um einen Abschnitt der Muffe neben sowohl dem distalen als auch dem proximalen Endabschnitt der Manschette freizulegen.

18. Verankerungsgerät nach Anspruch 1, wobei die Zugangsmuffe eine Kanüle ist, wobei:
die Manschette ein distales Ende und ein proximales Ende und ein Lumen zur gleitbaren Aufnahme der Kanüle umfasst;
die aufblasbare Membran ein radial expandierbares Element ist, das an das distale Ende der Manschette befestigt ist; wobei die Verankerungskanüle Folgendes umfasst:
eine Rückhaltemanschette (12, 410), die an das proximale Ende der Manschette befestigt ist; und
ein Eingriffselement (120, 222, 322), das zwischen der Kanüle und der Manschette angebracht ist, um die Bewegung der Kanüle mit Bezug auf die Manschette zu ermöglichen.

19. Verankerungsgerät nach Anspruch 18, wobei das Eingriffselement angeordnet ist, um den Durchgang von Fluid zwischen der Kanüle und der Manschette zu verhindern.

20. Verankerungsgerät nach Anspruch 1, wobei die Zugangsmuffe eine Kanüle ist, wobei:
die Manschette eine distale Verankerungsvorrichtung (401) ist, die gleitbar die Kanüle aufnimmt, wobei die distale Verankerungsvorrichtung eine expandierbare Muffe umfasst, die die aufblasbare Membran bereitstellt; die Verschlussvorrichtung zur Befestigung der Position der distalen Verankerungsvorrichtung mit Bezug auf die Kanüle dient; wobei das Verankerungsinstrument Folgendes umfasst:
eine proximale Verankerungsvorrichtung (402), die auf der Kanüle in einer Position proximal zur distalen Verankerungsvorrichtung angebracht ist.

21. Verankerungsgerät nach Anspruch 20, wobei die proximale Verankerungsvorrichtung eine Schaummanschette (402) umfasst.

22. Verankerungsgerät nach Anspruch 20, wobei die proximale Verankerungsvorrichtung gleitbar die Kanüle aufnimmt und weiter eine Verschlussvorrichtung umfasst, um die Position der proximalen Verankerungsvorrichtung mit Bezug auf die Kanüle zu befestigen.

23. Verankerungsgerät nach Anspruch 20, wobei die Kanüle ein Lumen definiert und weiter für ein Instrument geeignet ist, das in dem Lumen aufgenommen ist.

24. Verankerungsgerät nach Anspruch 20, wobei das Instrument einen Trokar (102) umfasst.

## Revendications

1. Appareil d'ancrage (100, 206, 306, 400, 500) pour l'utilisation avec un manchon d'accès (104, 506), le manchon d'accès étant adapté pour le passage à travers un tissu et possédant une lumière permettant l'introduction d'instruments à travers le manchon, l'appareil d'ancrage comprenant :
un collier (108, 208, 308, 404, 504) pour le positionnement sur le manchon d'accès, le collier définissant un axe longitudinal ;
une membrane gonflable (110, 210, 212, 310, 401, 402, 501, 502) fixée au collier, **caractérisé en ce que** :
le collier est un collier oblong annulaire contigu audit manchon d'accès, adapté pour un mouvement coulissant par rapport au manchon d'accès ;
la membrane gonflable entoure le collier et est fixée à celui-ci et peut s'agrandir pour entrer fermement en prise avec un tissu et pour ancrer sensiblement le collier par rapport au tissu tout en permettant le mouvement axial ou coaxial du manchon d'accès par rapport au collier ; et
un dispositif de verrouillage (512) fixe la position du collier par rapport au manchon.

2. Appareil d'ancrage selon la revendication 1, comprenant en outre un élément bague (112) monté de façon coaxiale sur une partie intermédiaire de la membrane gonflable et agencé pour exposer une partie de la membrane gonflable le long d'une extrémité du collier.

3. Appareil d'ancrage selon la revendication 2, dans lequel l'élément bague est sensiblement équidistant par rapport à une extrémité proximale et une extrémité distale du collier, et dans lequel la membrane gonflable est exposée le long des extrémités proximale et distale du collier oblong.

4. Appareil d'ancrage selon la revendication 1, dans lequel le manchon d'accès est une canule (104) et la membrane gonflable est un dispositif d'ancrage de ballonnet positionnable sur la canule, l'appareil d'ancrage pouvant coulisser par rapport à la canule ; et le dispositif de verrouillage fixe la position du dispositif d'ancrage de ballonnet par rapport à la canule.

5. Appareil d'ancrage selon la revendication 4, comprenant en outre un élément de prise (120, 222, 322) disposé entre la canule et le dispositif d'ancrage pour entrer en prise de façon coulissante avec le dispositif d'ancrage de ballonnet et la canule tout en permettant le mouvement de la canule et du dispositif d'ancrage de ballonnet par rapport à un ancrage.

6. Appareil d'ancrage selon la revendication 5, dans lequel l'élément de prise est agencé pour empêcher le passage de fluide entre la canule et le dispositif d'ancrage de ballonnet.

7. Appareil d'ancrage selon la revendication 4, dans lequel :
le collier est configuré et adapté pour recevoir la canule de façon coulissante ;
la membrane gonflable est un manchon configuré et adapté pour recouvrir le collier oblong, dans lequel une première extrémité du manchon est fixée à une extrémité distale du collier oblong et une seconde extrémité du manchon est fixée à une extrémité proximale du collier oblong.

8. Appareil d'ancrage selon la revendication 7, comprenant en outre :
une bague allongée (112) montée de façon coaxiale autour du manchon élastique ; et
un tube de gonflage (118, 218, 316, 408) en communication fluidique avec un espace défini entre le collier oblong et le manchon.

9. Appareil d'ancrage selon la revendication 8, dans lequel le collier oblong possède une longueur et dans lequel la bague allongée possède une longueur qui est plus courte que la longueur du collier oblong, dans lequel une partie du manchon est exposée au moins le long d'une extrémité du collier oblong.

10. Appareil d'ancrage selon la revendication 4, dans lequel :
le collier est configuré et adapté pour recevoir la canule de façon coulissante ;
la membrane gonflable est un ballonnet distal (210, 401, 501) fixé à une extrémité distale du collier oblong ;
et le dispositif d'ancrage de ballonnet comprend un ballonnet proximal (212, 502) fixé à une extrémité proximale du collier oblong.

11. Appareil d'ancrage selon la revendication 10, comprenant en outre un premier tube de gonflage (216) en communication fluidique avec le ballonnet proximal ; et
un second tube de gonflage (218) en communication fluidique avec le ballonnet distal par l'intermédiaire d'une lumière de gonflage formée à travers le collier oblong.

12. Appareil d'ancrage selon la revendication 11, dans lequel le collier oblong définit une lumière de gonflage (220, 320) en communication avec le tube de gonflage.

13. Appareil d'ancrage de ballonnet ajustable selon la revendication 10, dans lequel le ballonnet distal et le ballonnet proximal sont espacés l'un de l'autre afin de mettre un tissu en prise entre ceux-ci.

14. Appareil d'ancrage selon la revendication 4, dans lequel :
le collier est un collier oblong configuré et adapté pour entourer la canule ;
la membrane gonflable est un ballonnet fixé à une extrémité distale du collier oblong ; et
le dispositif d'ancrage comprend un tube de gonflage en communication fluidique avec le ballonnet par l'intermédiaire d'une lumière de gonflage formée à travers le collier oblong.

15. Appareil d'ancrage selon la revendication 14, comprenant en outre un collier de retenue (312, 402) possédant une ouverture à travers celui-ci pour le positionnement sur le collier oblong.

16. Appareil d'ancrage selon la revendication 1, qui est un dispositif d'ancrage pour l'utilisation avec un instrument chirurgical fournissant le manchon d'accès, l'instrument étant adapté pour l'accès percutané à travers le tissu, dans lequel :
le collier possède une partie d'extrémité distale, une partie intermédiaire et une partie d'extrémité proximale, le collier définissant une lumière pour le passage de l'instrument chirurgical à travers celle-ci ;
la membrane gonflable est un manchon fixé à une surface extérieure du collier, le manchon étant adapté pour s'agrandir dans une direction radiale pour entrer fermement en prise avec le tissu et ancrer sensiblement le collier par rapport au tissu tout en permettant le mouvement de l'instrument chirurgical par rapport au collier ; et
le dispositif de verrouillage est pour fixer la position du collier par rapport à l'instrument chirurgical.

17. Appareil d'ancrage selon la revendication 16, comprenant en outre un élément bague (112) monté de façon coaxiale sur la partie intermédiaire du collier et agencé pour exposer une partie du manchon près des extrémités distale et proximale du collier.

18. Appareil d'ancrage selon la revendication 1, dans lequel le manchon d'accès est une canule, dans lequel :
le collier possède une extrémité distale et une extrémité proximale et une lumière pour recevoir la canule de façon coulissante ;
la membrane gonflable est un élément pouvant s'agrandir de de façon radiale fixé à l'extrémité distale du collier ; la canule d'ancrage comprend :
un collier de retenue (12, 410) fixé à l'extrémité proximale du collier ; et
un élément de prise (120, 222, 322) disposé entre la canule et le collier pour permettre le mouvement de la canule par rapport au collier.

19. Appareil d'ancrage selon la revendication 18, dans lequel l'élément de prise est agencé pour empêcher le passage de fluide d'entre la canule et le collier.

20. Appareil d'ancrage selon la revendication 1, dans lequel le manchon d'accès est une canule, dans lequel :
le collier est un dispositif d'ancrage distal (401) recevant la canule de façon coulissante, le dispositif d'ancrage distal comprenant un manchon pouvant s'agrandir qui fournit la membrane gonflable ; le dispositif de verrouillage est pour fixer la position du dispositif d'ancrage distal par rapport à la canule ;
l'instrument d'ancrage comprenant :
un dispositif d'ancrage proximal (402) disposé sur la canule dans une position proximale par rapport au dispositif d'ancrage distal.

21. Appareil d'ancrage selon la revendication 20, dans lequel le dispositif d'ancrage proximal comprend un collier en mousse (402).

22. Appareil d'ancrage selon la revendication 20, dans lequel le dispositif d'ancrage proximal reçoit la canule de façon coulissante et comprend en outre un dispositif de verrouillage pour fixer la position du dispositif d'ancrage proximal par rapport à la canule.

23. Appareil d'ancrage selon la revendication 20, dans lequel la canule définit une lumière et est en outre appropriée pour un instrument reçu dans la lumière.

24. Appareil d'ancrage selon la revendication 20, dans lequel l'instrument comprend un trocart (102).
